(19) Europäisches Patentamt
European Patent Office
Office européen des brevets

(11) Publication number: **0 212 745 B1**

# (12) EUROPEAN PATENT SPECIFICATION

(45) Date of publication of patent specification: **10.04.91** (51) Int. Cl.5: **A61K 9/54**, A61K 31/19

(21) Application number: **86201388.5**

(22) Date of filing: **07.08.86**

## (54) Drug particles having constant release.

(30) Priority: **16.08.85 US 766745**

(43) Date of publication of application:
**04.03.87 Bulletin 87/10**

(45) Publication of the grant of the patent:
**10.04.91 Bulletin 91/15**

(84) Designated Contracting States:
**BE CH DE FR IT LI LU NL**

(56) References cited:
**EP-A- 0 080 341**
**EP-A- 0 092 060**
**EP-A- 0 094 123**
**WO-A-85/03437**
**GB-A- 2 151 921**

(73) Proprietor: **THE PROCTER & GAMBLE COMPANY**
**One Procter & Gamble Plaza**
**Cincinnati Ohio 45202(US)**

(72) Inventor: **Kelm, Gary Robert**
**8524 Althaus Road**
**Cincinnati Ohio 45247(US)**
Inventor: **Keim, Richard Edward**
**2063 Waycross Road**
**Cincinnati Ohio 45240(US)**

(74) Representative: **L'Helgoualch, Jean**
**Cabinet Sueur et L'Helgoualch 78, rue Carnot**
**F-95240 Cormeilles-en-Parisis(FR)**

EP 0 212 745 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

The present invention is related to sustained release drug particles having a release profile providing for more constant release of the drug active.

### BACKGROUND OF THE INVENTION

This invention relates to pharmaceutical particles providing a prolonged release of the drug active.

The administration of drugs orally oftentimes presents serious problems. In very many cases, it is necessary for the drugs to be absorbed from the gastro-intestinal tract into the bloodstream. When a drug is given by mouth, the assimilation is normally fairly rapid and the level in the blood reaches a maximum. Then it declines as the drug is excreted or otherwise removed from the bloodstream. The blood level falls and finally reaches a level so low that it is no longer effective and it is then necessary to take another dose. The peaks and valleys thus caused in blood level have several disadvantages. One is the necessity of taking frequent small doses in order to maintain a therapeutically desired blood level. This is awkward and presents a problem as the patient has to continue to take further doses. A second drawback is that in order to have a therapeutically useful blood level, each dose has to be fairly large so that after the blood level peak is reached and begins to subside, there will still remain sufficient of the drug to be useful. The necessity for large doses brings with it some problems. The large initial doses may be unacceptable or may produce some undesirable side reactions.

For the reasons set out above, various attempts have been made to slow up the release of drugs taken orally, producing a sustained release product. Sometimes it is felt desirable only to slow up the release sufficiently so that it extends over a long enough time to even out the more marked peaks and valleys in blood level. In other cases, it may be desirable to prevent assimilation from the stomach but to permit assimilation from the intestine. Various coatings have been developed (i.e., enteric coatings) to achieve this purpose.

Some of the attempts at making improved sustained-release particles are reflected in patents issued in the area. One patent is U.S. 3,078,216, February 19, 1963 to Greif, which discloses sustained-release particles having a coating of water-insoluble wax and possibly, a second enteric coating. U.S. Patent in, 459,279, July 10, 1984 to Stricker et al., discloses drug granules having a coating of a water insoluble component and a water soluble component. As a final second coating an enteric polymer may be employed. European Patent Application 0 061 217, September 29, 1982 to Giudice et al., discloses drug particles having an inert core, a first layer of the drug and an outer, second, layer of polyvinyl pyrrolidone. European Patent Application 0 092 060, October 26, 1983 to Colombo et al., discloses drug particles having an enteric polymer in the drug core, a first coating designed to regulate the release of the drug and a second coating to protect the first coating.

EP-A- 0 080 341 discloses controlled release multiple-units formulations wherein the individual units comprise cross-sectionally substantially homogenous cores containing particles of an active substance, the cores being coated with a coating which is substantially resistant to gastric conditions, but which is erodable under the conditions in the small intestine.

WO-A- 8 500 006 discloses controlled release multiple-units formulations, wherein the individual units contain an active substance and are coated with a substantially water-insoluble, but water diffusible controlled release coating including an inner film of a water-dispersible film forming agent and a polymeric substance to impart compressibility to the coating and an optional outer film layer comprising a film-forming agent to prevent adhesion between the units and impart flowability to the units. EP-A- 0 094 123 discloses enteric coated therapeutic granules which have a first coating of a therapeutic agent over the therapeutic core and an enteric coating adhered to the therapeutic coating.

While the above described references disclose delayed release drug particles, they as well as other prior art executions, do not provide complete answers to making totally satisfactory delayed release products, particularly those containing propionic acid derivatives.

Some drugs, such as ibuprofen do not appear to follow a pattern of constant release found for other drugs. Instead, the dissolution data for ibuprofen particles having a porous coating indicate that the controlling surface area is not the constant surface area of the porous coating, but the surface area of the ibuprofen particle which decreases with time as the drug dissolves. Thus, the dissolution rate decreases with time.

The decrease in dissolution rate with time of an ibuprofen particle having a porous coating can be compensated for if the resistance of the porous coating to diffusion also decreases with time. This can be achieved by decreasing its thickness with time. The present invention provides a coating on an ibuprofen particle that decreases in thickness at a rate such that the decrease in surface area of the ibuprofen particle as it dissolves is compensated for and the dissolution rate remains essentially constant.

It is a further object of the present invention to provide an analgesic effective against a variety of pain, acute and chronic.

It is still a further object of the present invention to provide delayed release particles which are effective in the treatment of dysmenorrhea.

These and other objects will become readily apparent from the detailed description which follows.

As used herein all percentages and ratios are by weight unless otherwise specified.

## SUMMARY OF INVENTION

The present invention relates to therapeutic particles containing a drug active core, an inner coating of an enteric material and an outer coating of a material which is insoluble at any pH and acts as a diffusion barrier for the drug active.

## DETAILED DESCRIPTION OF THE INVENTION

The essential as well as optional components of the present particles are described below. In the present application the following terms have the meanings given.

"Pharmaceutically-acceptable" or "pharmacologically-acceptable", as used herein, means that the ingredients used in the compositions are suitable for use in contact with the tissue of humans , without undue toxicity , irritation, allergic response, and the like, commensurate with a reasonable benefit/risk ratio.

The term "comprising", as used herein, means that various other compatible components, including both active and inert ingredients, can be conjointly employed in the compositions of this invention. The term "comprising" thus encompasses and includes the more restrictive terms "consisting of" and "consisting essentially of".

By "compatible" herein is meant that the components of the present invention are capable of being commingled without interacting in a manner which would substantially decrease the efficacy of the therapeutic under ordinary use conditions. Drug Active

The drug actives found useful in the particles of the present invention are propionic acid derivatives. Such compounds have both analgesic and anti-inflammatory activity and can be used at appropriate dosage levels in the present invention. The compounds are frequently used in their free acid form but also can be used in the form of their pharmaceutically accepted salts.

The propionic acid derivatives for use herein include, but are not limited to, ibuprofen, flurbiprofen, fenoprofen, ketoprofen, indoprofen, suprofen, and fluprofen. Structurally related propionic acid derivatives having similar analgesic and anti-inflammatory properties are also intended to be encompassed by this group. Structural formulas for representative group members are set forth below:

## Propionic Acid Derivatives

ibuprofen

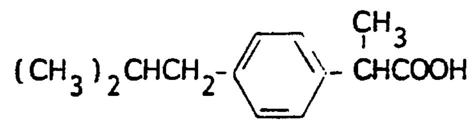

flurbiprofen

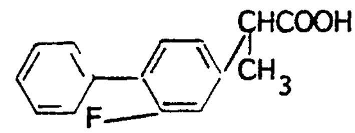

fenoprofen

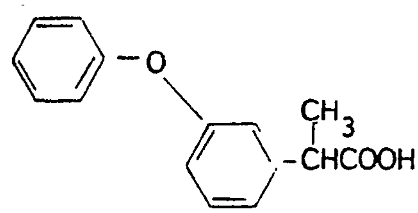

indoprofen

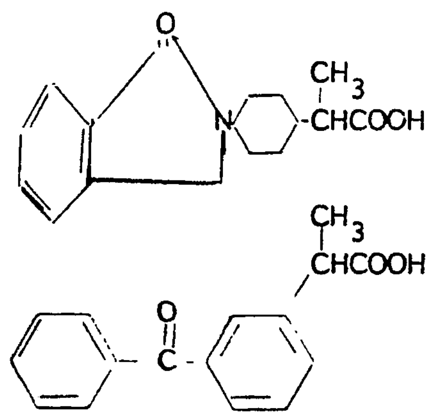

ketoprofen

fluprofen

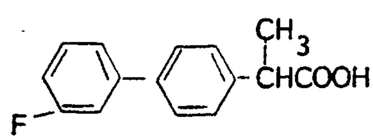

Thus, "propionic acid derivatives" as defined herein are non-narcotic analgesics/nonsteroidal anti-inflammatory drugs having a free --$CH(CH_3)COOH$ or --$CH_2CH_2COOH$ group (which optionally can be in the form of a pharmaceutically acceptable salt group, e.g., --$CH(CH_3]COO\text{-}Na^+$ or --$CH_2CH_2COO\text{-}Na^+$), typically attached directly or via a carbonyl function to a ring system, preferably to an aromatic ring system.

A drug most preferred for use in the present compositions is ibuprofen.

The drug active can form the entire core of the particles of the present invention or be combined with other pharmaceutically acceptable materials such as polysorbate 80 (POE [20] sorbitan monooleate] and crosslinked polyvinyl pyrrolidones (e.g., PVP-XL] and other disintegrants. These materials are used at a level of from 0% to 2% based on the total weight of the core. The size of the core composition can be chosen to meet the formulator's needs but is preferably from act 0.1 mm to

2mm, most preferably from 0.5mm to 1.2 mm.

Inner Coating

The inner coating used on the present particles is an enteric polymer. The polymer is selected from the group consisting of acrylic polymers and copolymers. The material should not be soluble in the stomach (i.e., acidic pH environment) but be soluble in the intestines. A preferred material is Eudragit™ L30D offered by Rohm Pharma. This material is a copolymer, anionic in character based on polymethacrylic acid and acrylic acid esters having the following repeating structure:

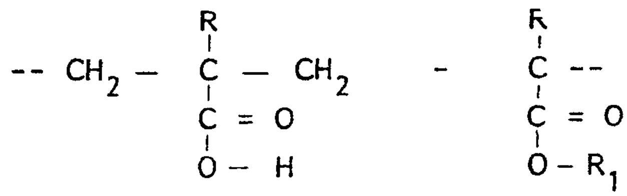

R = H or $CH_3$; $R_1$ = $CH_3$ or $C_2H_5$

The L30D material has a mean molecular weight of about 250,000.

The inner coating is preferably applied to the core so that the weight of coating solids is from 1% to 30% based on the total weight of the core plus coating solids, preferably from 2% to 20%.

Materials which modify the enteric coating can be used with the enteric. Such materials include pharmaceutically acceptable plasticizers such as triacetin which are compatible with the drug active. Plasticizers are used at a level of from 0% to 25%, preferably from 0% to 15% based on the weight of the solids in the first coating.

## Outer Coating

The outer coating on the present particles serves as a diffusion barrier for the drug active. The coating material is a polymer or copolymer based on methacrylic acid. The material should be permeable to water and solubilized drugs but is not soluble at any of the pHs found in the stomach or the intestines. A preferred material is Eudragit™E 30D offered by Rohm Pharma. This material is a copolymer, neutral in character, based on poly(meth)acrylic acid esters having the following repeating structure:

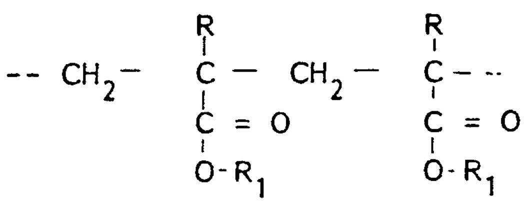

R = H or $CH_3$; $R_1$ = $CH_3$ or $C_2H_5$

Eudragit™ E-30D has a mean molecular weight of about 800,000

Materials which can be used as a partial replacement of Eudragit E-30D are copolymers synthesized from acrylic and methacrylic acid esters with a low content of quaternary ammonium groups. The repeating structure is as follows:

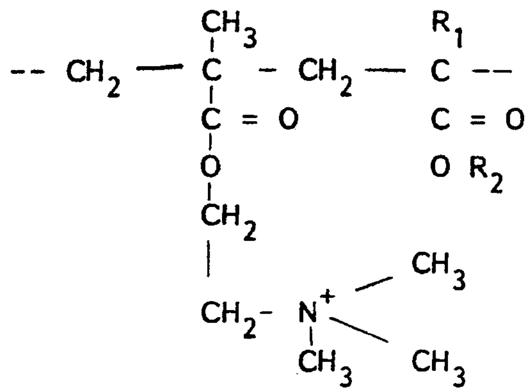

$R_1$ = H or $CH_3$; $R_2$ = $CH_3$ or $C_2H_5$

The molar ratio of the ammonium groups to the remaining meth(acrylic) acid esters is 1:20 with a material identified as Eudragit™RL and 1:40 with a material identified as Eudragit™ RS.

As with Eudragit™ E-30D material, the RL and RS materials are permeable to water and to the drug actives but are virtually insoluble at any pH.

As with the inner coating, the outer coating may contain pharmaceutically acceptable materials which modify the coating. Such materials include hydroxypropyl methyl cellulose, talc, sodium chloride, and polysorbate 80 (POE [20] sorbitan monooleate]. These materials are conventional adjuvant materials for use with coatings and can be used at levels found by the formulator necessary to give the effect desired (e. g., hydroxypropyl methyl cellulose up to 8% of the acrylic polymer; talc up to 35% of the acrylic polymer; polysorbate 80 up to 4% of the acrylic polymer; and sodium chloride up to act 10% of the acrylic polymer).

The outer coating is used in the present particles at a level such that the coating is from 2% to 20% of the total solids in the core, the inner coating and the outer coating, preferably from 4% to 8%. The amount of acrylic polymer in the outer coating, on this same basis, should be from 1.2% to 11.8% preferably from 2.4% to 4.7%.

## METHOD OF MANUFACTURE

A method for manufacturing the particles of the present invention is given in Example 1.

## FIELD OF USE

The particles of the present invention can be used in tablets, capsules or any other convenient form. As indicated previously, the drug actives are known for providing relief from a variety of pain.

The following examples further describe preferred embodiments within the scope of the present invention.

## EXAMPLES I, II & III

The following are representative particles of the present invention.

| | Component | Weight % I | II | III |
|---|---|---|---|---|
| Core | Ibuprofen | 89.3 | 89.5 | 88.4 |
| | Polysorbate 80 | -- | 0.6 | -- |
| | PVP XL | -- | 0.5 | -- |
| Inner Coating | Eudragit™ L 30D | 5.7 | 5.4 | 6.9 |
| | Triacetin | -- | -- | 0.7 |
| Outer Coating | Eudragit™ E 30D | 5.0 | 4.0 | 2.4 |
| | Talc | -- | -- | 1.58 |
| | Polysorbate 80 | -- | -- | 0.02 |
| | | 100.0 | 100.0 | 100.00 |

The particles described above are prepared by first compacting the ibuprofen at 793kPa (115 psi) to 1344kPa to (195 psi), preferably from 1000kPa (145) to 1138kPa (165 psi), in a roller compactor. The compaction is then milled and the resulting particles sieved to obtain particles in the range of 0.1mm to 2.0mm, preferably from 0.5mm to 1.2mm.

The cores are then coated with the inner, Eudragit™ L 30D, coating followed by the Eudragit™ E 30D coating. An inner coating dispersion is prepared by diluting the coating solids with water to obtain a dispersion having about 16.5% non-volatiles. The uncoated cores are then introduced into a Wurster fluidized bed coater using fluidizing air having a temperature of from about 35 tv 60°C. The coating dispersion is next added to the coater by means of a peristaltic pump. The atomizing and fluidizing air flow rates and coating dispersion are adjusted to maintain a coating temperature of 25 to 40°C.

The cores with the inner coating are subsequently coated with a dispersion of the Eudragit™ E 30D. The dispersion having a solids concentration of about 20% is introduced into the coater using the same conditions used to apply the first coating. The final coated particles are dried at elevated temperatures for a period of hours.

The coated particles exhibit excellent release of the ibuprofen over a period of many hours.

## Claims

**1.** Drug particles comprising:

(a) a propionic acid derivative core comprising non-narcotic analgesics/nonsteroidal anti-inflammatory drugs having a free --CH ($CH_3$) COOH or --$CH_2CH_2COOH$ group (which optionally can be in the form of a pharmaceutically acceptable salt group), typically attached directly or via a carbonyl function to a ring system;

(b) an inner coating of an enteric material selected from the group consisting of acrylic polymers and copolymers; and

(c) an outer coating of a material permeable to water and solubilized drugs, and insoluble in the stomach and intestines selected from the group consisting of methacrylic acid polymers and copolymers.

**2.** Drug particles according to Claim 1 wherein the propionic acid derivative is selected from the group consisting of ibuprofen, indoprofen, flurbiprofen, fenoprofen, fluprofen, ketoprofen and suprofen.

**3.** Drug particles according to Claim 2 wherein the inner coating is present at a level of from 1% to 30% of the total weight of the core and the inner coating.

4. Drug particles according to Claim 3 wherein the outer coating is present at a level of from 2% to 20% of the total weight of the core, the inner coating and the outer coating.

5. Drug particles according to Claim 4 wherein the propionic acid derivative is ibuprofen.

6. Drug particles according to Claim 5 wherein the inner coating is based on polymethacrylic acid and acrylic acid esters and has a mean molecular weight of about 250,000.

7. Drug particles according to Claim 6 wherein the outer coating is based on polymethacrylic acid esters and has a mean molecular weight of about 800,000.

## Revendications

1. Particules de médicament comprenant:
   (a) un noyau de dérivé d'acide propionique comprenant des médicaments analgésigues non-narcotiques/anti-inflammatoires non-stéroïdaux ayant un groupe libre $-CH(CH_3)COOH$ ou $-CH_2CH_2COOH$ (qui peut éventuellement être sous la forme d'un sel pharmaceutiquement acceptable) , attaché de manière caractéristique à un système cyclique directement ou par l'intermédiaire d'une fonction carbonyle;
   (b) un enrobage interne d'un matériau gastro-résistant choisi dans le groupe constitué par les polymères et copolymères acryliques; et
   (c) un enrobage externe d'un matériau perméable a l'eau et aux médicaments solubilisés, et insoluble dans l'estomac et les intestins, choisi dans le groupe constitué par les polymères et les copolymères d'acide méthacrylique.

2. Particules de médicament selon la revendication 1, caractérisées en ce que le dérivé d'acide propionique est choisi dans le groupe constitué par l'ibuprofène, l'indoprofène, le flurbiprofène, le fénoprofène, le fluprofène, le kétoprofène et le suprofène.

3. Particules de médicament selon la revendication 2, caractérisées en ce que l'enrobage interne représente de 1% à 30% du poids total du noyau et de l'enrobage interne.

4. Particules de médicament selon la revendication 3, caractérisées en ce que l'enrobage externe représente de 2% à 20% du poids total du noyau, de l'enrobage interne et de l'enrobage externe.

5. Particules de médicament selon la revendication 4, caractérisées en ce que le dérivé de l'acide propionique est l'ibuprofène.

6. Particules de médicament selon la revendication 5, caractérisées en ce que l'enrobage interne est basé sur un polymère d'acide méthacrylique et d'esters d'acide acry lique et présente un poids moléculaire moyen d'environ 250 000.

7. Particules de médicament selon la revendication 6, caractérisées en ce que l'enrobage externe est basé sur un polymère d'esters d'acide méthacrylique et présente un poids moléculaire moyen d'environ 800 000.

## Ansprüche

1. Wirkstoffpartikel, umfassend:
   (a) einen Propionsäurederivat-Kern, enthaltend nicht narkotische Schmerzmittel/nicht steroide entzündungshemmende Wirkstoffe mit einer freien $--CH(CH_3)COOH$- oder $-CH_2CH_2COOH$-Gruppe (welche wahlweise in Form einer pharmazeutisch zulässigen Salzgruppe vorliegen kann), die typischerweise direkt oder via einer Carbonylfunktion an ein Ringsystem gebunden ist,
   (b) einen inneren überzug aus einem enterischen Material, ausgewählt aus der Gruppe, bestehend aus Acrylpolymeren und -copolymeren, und
   (c) einen äußeren Überzug aus einem Material, das für Wasser und gelöste Wirkstoffe durchlässig

ist und im Magen und Darm unlöslich ist, ausgewählt aus der Gruppe, bestehend aus Methacrylsäurepolymeren und -copolymeren.

2. Wirkstoffpartikel gemäß Anspruch 1, worin das Propionsäurederivat ausgewählt ist aus der Gruppe, bestehend aus Ibuprofen, Indoprofen, Flurbiprofen, Fenoprofen, Fluprofen, Ketoprofen und Suprofen.

3. Wirkstoffpartikel gemäß Anspruch 2, worin der innere Überzug in einer Menge von 1 bis 30%, bezogen auf das Gesamtgewicht aus Kern und innerem Überzug, vorhanden ist.

4. Wirkstoffpartikel gemäß Anspruch 3, worin der äußere überzug in einer Menge von 2 bis 20%, bezogen auf das Gesamtgewicht aus Kern, innerem Überzug und äußerem Überzug, vorhanden ist.

5. Wirkstoffpartikel gemäß Anspruch 4, worin das Propionsäurederivat Ibuprofen ist.

6. Wirkstoffpartikel gemäß Anspruch 5, worin der innere Überzug auf Polymethacrylsäure und Acrylsäureestern beruht und ein mittleres, Molekulargewicht von etwa 250 000 hat.

7. Wirkstoffpartikel gemäß Anspruch 6, worin der äußere Überzug auf Polymethacrylsäureestern beruht und ein mittleres Molekulargewicht von etwa 800 000 hat.